# EUROPEAN PATENT SPECIFICATION

(11) **EP 4 573 182 B1**
(45) Date of publication and mention of the grant of the patent: **21.10.2026**
(21) Application number: 23790753.0
(22) Date of filing: 19.08.2023
(51) Int. Cl.: C12N 1/20, C12N 1/205, A01N 63/20, C12R 1/085, C12R 1/38

(54) **BACTERIAL COMPOSITION, AN INOCULUM FOR BRASSICACEAE PLANTS BIOTIZATION CONTAINING THE SAID COMPOSITION AND BACTERIAL STRAINS CONTAINED THEREIN**
BAKTERIENZUSAMMENSETZUNG, EIN INOKULUM FÜR DIE BIOTISIERUNG VON BRASSICACEAE-PFLANZEN ENTHALTEND DIE ZUSAMMENSETZUNG UND DARIN ENTHALTENE BAKTERIENSTÄMME
COMPOSITION BACTÉRIENNE, INOCULUM POUR LA BIOTISATION DE PLANTES BRASSICACEAE CONTENANT LADITE COMPOSITION ET SOUCHES BACTÉRIENNES CONTENUES DANS CELLE-CI

(30) Priority: 19.08.2022 PL 44204522
(43) Date of publication of application: 25.06.2025
(73) Proprietor: Uniwersytet Jagiellonski, 31-007 Krakow (PL); Uniwersytet Rolniczy im. Hugona Kollataja w Krakowie, 31-120 Krakow (PL)
(72) Inventor: ROZPADEK, Piotr, 32-566 Poreba Zegoty (PL); WAZNY, Rafal, 30-629 Kraków (PL); JEDRZEJCZYK, Roman J., 31-952 Kraków (PL); DOMKA, Agnieszka, 30-389 Kraków (PL); KALISZ, Andrzej, 30-667 Kraków (PL)
(74) Representative: Witek, Rafal
(86) International application number: PCT/PL2023/050069
(87) International publication number: WO 2024/039254

(56) References cited:
- WO-A1-2014/145883
- WO-A1-2020/214290
- SUNG-HEUN CHO ET AL: "Four new species of Chryseobacterium from the rhizosphere of coastal sand dune plants, Chryseobacterium elymi sp. nov., Chryseobacterium hagamense sp. nov., Chryseobacterium lathyri sp. nov. and Chryseobacterium rhizosphaerae sp. nov.", SYSTEMATIC AND APPLIED MICROBIOLOGY, vol. 33, no. 3, 1 April 2010 (2010-04-01), AMSTERDAM, NL, pages 122 - 127, XP055558577, ISSN: 0723-2020, DOI: 10.1016/j.syapm.2009.12.004
- PASSERA ALESSANDRO ET AL: "Characterization of Lysinibacillus fusiformis strain S4C11: In vitro, in planta, and in silico analyses reveal a plant-beneficial microbe", MICROBIOLOGICAL RESEARCH, FISCHER, JENA, DE, vol. 244, 5 December 2020 (2020-12-05), XP086454468, ISSN: 0944-5013, [retrieved on 20201205], DOI: 10.1016/J.MICRES.2020.126665
- CARD STUART D ET AL: "Beneficial endophytic microorganisms of Brassica- A review", BIOLOGICAL CONTROL, SAN DIEGO, CA, US, vol. 90, 6 June 2015 (2015-06-06), pages 102 - 112, XP029278733, ISSN: 1049-9644, DOI: 10.1016/J.BIOCONTROL.2015.06.001
- GUSTAB MACIEJ ET AL: "Beneficial impact of multi-bacterial inoculation on growth of selected Brassicaceae seedlings in a greenhouse culture", SCIENTIA HORTICULTURAE, 27 October 2023 (2023-10-27), pages 112575, XP093107351, Retrieved from the Internet <URL:https://pdf.sciencedirectassets.com/271177/1-s2.0-S0304423823X00144/1-s2.0-S0304423823007434/main.pdf?X-Amz-Security-Token=IQoJb3JpZ2luX2VjEJL//////////wEaCXVzLWVhc3QtMSJHMEUCIHzyFreDTlGWuUtbbHryE3tPYHocm5koRqZ5VMkAXNekAiEAs6QwLpzF4DC8152Kd402pfjO/ZGctlf67UX4fESx8A0quwUI6///////////ARAFGgwwNTkwMDM1N> [retrieved on 20231130], DOI: 10.1016/j.scienta.2023.112575

## Description

### Technical field

The subject matter of the invention is an inoculum to enhance Brassicaceae plant growth parameters containing microbial strains: *Chryseobacterium lathyri* with assigned reference No. B/00412 (int. UNIJAG.PL.OP280), *Lysinibacillus fusiformis* with assigned reference No. B/00414 (int. UNIJAG.PL.OP290), *Bacillus cereus* with assigned reference No. B/00413 (int. UNIJAG.PL.OP287), and *Pseudomonas protegens* with assigned reference No. B/00415 (int. UNIJAG.PL.OP300). The inoculum disclosed herein can be used for biotization of Brassicaceae plants and for production of fully developed plant seedlings, e.g. for their direct sale.

### Background art

Efforts to obtain biopreparations that enhance qualitative parameters, as well as a biomass of edible plants are important from both the economic and utilitarian standpoints. Current efforts also include studying the effect of these preparations on increasing the yield and quality of Brassicaceae plants, which hold a large share of the vegetable market, as described by Paweda et al., who observed a positive effect of co-inoculation with *Trichoderma* fungus and arbuscular mycorrhizal fungi (AMF). [1] The vegetable market is a growing and important source of food for human consumption, which justifies the outlook for new stimulants to replace products based on inorganic salts that often contain heavy metal impurities. In Brassicaceae plant production, it is common to use ready-made seedlings available in the market. High-quality substrates and optimal conditions, such as temperature, humidity, light intensity, and color, are essential for Brassicaceae seedling production. Ensuring young plants receive the right amount of nutrients through fertilization is an important factor. The quality of a seedling is crucial for successful incorporation into the soil, as weak seedlings are more challenging to root and subsequently yield less. Currently, the production of Brassicaceae seedlings involves multi-stage fertilization using compound preparations and root growth activators. It is therefore noteworthy that replacing stimulants based on inorganic salts with bioproducts is environmentally beneficial and aligns with sustainable development principles.

US Patent No. US 7.232.565 B2, "Use of endophytic fungi to treat plants" describes a method for plant inoculation, including Brassicaceae plants, with endophytic fungi, especially *Curvularia,* to enhance plant tolerance to drought, increased content of metals, and high temperatures. This patent is distinct from the present one as it utilizes fungi as microorganisms instead of bacteria, and its research objectives differ. Furthermore, plants used in research cited in the current description were not utilized in the aforementioned patent.

US Patent No. US 7.259.004 B1, "Endophytic Streptomycetes from higher plants with biological activity" details the utilization of an endophytic bacterial strain for producing chemicals that bolster a plant's defense against both microbial and plant pathogens. Both the bacterial strain and its application method diverge from this description.

US Patent No. US 10556839 B2, "Methods and Compositions for Improving Plant Traits" outlines a technique for enhancing nitrogen fixation in non-legume plants through bacteria. *In planta,* bacteria account for 1% or more of the plant's fixed nitrogen. The method of employing these bacteria differs significantly from the current description.

"Growth promotion of canola (rapeseed) seedlings by a strain of Pseudomonas putida under gnotobiotic conditions" (Can. J. Microbiol. vol. 33: p. 390-395) describes a method of inoculating canola (*Brassica campestris*) seeds with the nitrogen-fixing *Pseudomonas putida* (GR 12-2) strain. The said method comprises soaking superficially sterilized seeds in a bacterial suspension for 60 min, followed by sterile sowing into the substrate. Plants were cultivated under sterile conditions. In seedlings cultivated under sterile conditions, inoculation led to statistically significant root length increases. Inoculation with bacterial strains incapable of nitrogen fixation did not impact root length. The cited publication's inoculation method, bacterial strain, and cultivation procedure (sterile conditions) differ from this description.

"Effect of bacteria on the biology of diamondback moth (Plutella xylostella) on cabbage (Brassica oleraceae VAR. Capitata) cv. Midori" (Anais da Academia Pernambucana de Ciência Agronômica, Recife, vol. 2, p. 204-212, 2005) explores bacterial inoculation in Brassicaceae plant seeds. The publication aimed at investigating the potential of various growth-promoting rhizobacteria for biologically controlling *P. xylostella* (diamondback moth) in Midori cabbage during its vegetation phase. The bacteria used, the method of inoculation, and the study's objectives diverge from this application. In addition, using the bacteria described in this publication in different plant species or at varying times (at different inoculation times) might not replicate the outcomes as described by the authors and could even prove detrimental.

WO 2014/145883 describes an inoculum comprising, among others, Bacillus cereus strains, which is used for stimulating plant growth, such as growth of Brassicaceae.

WO 2020/214290 describes a composition comprising a Pseudomonas protegens strain and Bacillus species, which is used to improve the yield of a plant, such as Brassicaceae,

The purpose of the invention is to provide a novel method for producing an inoculum for Brassicaceae plant biotization, preferably Mizuna ( *Brassica rapa L. subsp. nipposinica* (L. H. Bailey) Hanelt), green cauliflower ( *Brassica oleracea L. convar. botyris* (L.) Alef. *var. botrytis cv.* 'Verde di Macerata'), kale ( *Brassica oleracea* L. var. *sabellica* L. *cv.* 'Halbhoher Gruner Krauser'), Chinese cabbage ( *Brassica rapa* L. subsp. *chinensis* (L.) Hanelt), napa cabbage ( *Brassica rapa* L. var. *pekinensis* (Lour.) Hanelt cv. 'Manoko F1'), white cabbage ( *Brassica oleracea L. convar. capitata* (L.) Alef. *var. alba* DC.), and red cabbage ( *Brassica oleracea L. convar. capitata* (L.) Alef. *var. rubra* (L.) Thell.), and the use of the inoculum in the cultivation process by the time seedlings are obtained, employing isolated and selectively chosen microbes, preferably *Chryseobacterium lathyri, Lysinibacillus fusiformis, Bacillus cereus,* and *Pseudomonas protegens.* The biopreparation significantly enhances plant growth, increasing the dry and fresh mass of the green parts and roots. This beneficial effect was observed under both non-fertilization and plant fertilization conditions. Concurrent fertilization and biotization of plants yield more advantageous results than fertilization alone in terms of obtained plant biomass. The developed biopreparation can be effectively employed in the industrial production of Brassicaceae vegetable seedlings as an agent to either reduce or complement additional fertilization.

### Summary of the Invention

The subject matter of the invention is a bacterial composition comprising the following strains: *Chryseobacterium lathyri* deposited in PCM under B/00412 (int. UNIJAG.PL.OP280), *Lysinibacillus fusiformis* deposited in PCM under B/00414 (int. UNIJAG.PL.OP290), *Bacillus cereus* deposited in PCM under B/00413 (int. UNIJAG.PL.OP287), and *Pseudomonas protegens* deposited in PCM under B/00415 (int. UNIJAG.PL.OP300).

The subject matter of the invention also pertains to an inoculum for Brassicaceae plant biotization containing the composition as defined above.

Preferably, the inoculum according to the invention is intended for accelerating the growth of Brassicaceae plants, more preferably for increasing the matter gain of the green parts or roots of the plants.

Preferably, the inoculum according to the invention is in the form of an aqueous bacterial suspension.

The subject matter of the invention is also a bacterial strain selected from: *Chryseobacterium lathyri* deposited in PCM under B/00412 (int. UNIJAG.PL.OP280), *Lysinibacillus fusiformis* deposited in PCM under B/00414 (int. UNIJAG.PL.OP290), *Bacillus cereus* deposited in PCM under B/00413 (int. UNIJAG.PL.OP287), and *Pseudomonas protegens* deposited in PCM under B/00415 (int. UNIJAG.PL.OP300).

All microbial strains according to the invention were deposited in accordance with the Budapest Treaty in the Polish Collection of Microorganisms (PCM) at the Institute of Immunology and Experimental Therapy of the Polish Academy of Sciences, ul. Rudolfa Weigla 12, 53-114 Wroclaw, Poland.

The designation of strains according to the invention is listed in Table 1 below.

**Table 1. Microbial strains according to the invention**

| Species | Internal strain designation | Shortened designation (see Figures) | PCM accession number |
|---|---|---|---|
| *Chryseobacterium lathyri* | UNIJAG.PL.OP280 | OP280 | B/00412 |
| *Lysinibacillus fusiformis* | UNIJAG.PL.OP290 | OP290 | B/00414 |
| *Bacillus cereus* | UNIJAG.PL.OP287 | OP287 | B/00413 |
| *Pseudomonas protegens* | UNIJAG.PL.OP300 | OP300 | B/00415 |

### Detailed Description of the Invention

The first aspect of the invention is therefore the inoculum and method for preparation of the inoculum for plant biotization by combining four bacterial strains: *Chryseobacterium lathyri, Lysinibacillus fusiformis, Bacillus cereus,* and *Pseudomonas protegens.*

Another aspect of the invention is plant biotization using the inoculum in seedling cultivation conditions.

In carrying out the first aspect of the invention, isolated, purified, identified, and deposited in relation to this application, pure bacterial cultures are grown separately in a liquid bacterial culture medium with shaking at 120-220 rpm until an optical density of 1-2 is reached, centrifuged, the precipitate is washed with saline by discarding the supernatant, the washed precipitate is suspended in saline, the obtained suspensions are combined, and the final solution is diluted 5-12x.

In carrying out the second aspect of the invention, a plant is inoculated in soil upon sowing into a substrate with 0.2-2 mL of inoculum/plant, and the treatment is repeated 5-15 days after sowing. The inoculum is suitable for use in soil substrates having a pH of 4.0-8.0. The inoculum can be added to a sterile or non-sterile substrate.

In a preferred embodiment of the inventive method, the liquid culture medium is a sterile nutrient agar (NA) with the following composition: peptone 5 g/L, meat extract 3 g/L, and agar 15 g/L.

In a preferred embodiment of the inventive method, the bacterial cultures are shaken at 180 rpm.

In a preferred embodiment of the inventive method, the optical density (OD600) of the cultures is 1.8 ±0.4.

In a preferred embodiment of the inventive method, the bacterial cultures are centrifuged for 5 min at 5000g.

In a preferred embodiment of the inventive method, the bacterial precipitate is washed twice. In a preferred embodiment of the inventive method, the bacterial precipitate is washed with a 0.9% aqueous NaCl solution.

In a preferred embodiment of the inventive method, the washed bacterial precipitate is suspended in 40 ml of a 0.9% aqueous NaCl solution.

In a preferred embodiment of the inventive method, the suspended culture is mixed and filled up to 1 L with a 0.9% aqueous NaCl solution.

In a preferred embodiment of the inventive method, the resulting bulk suspension is used for plant inoculation at a volume of 1 ml of inoculum/plant.

In a preferred embodiment of the inventive method, the plants are inoculated in soil twice.

In a preferred embodiment of the inventive method, the first dose of inoculum is administered on the day of sowing.

In a preferred embodiment of the inventive method, the second dose of inoculum is administered 7-10 days after the first dose.

In a preferred embodiment of the inventive method, the inoculum is applied to a soil substrate with a pH of 5.5.

In a preferred embodiment of the inventive method, plants are inoculated with the inoculum upon being transferred from multi-pots to larger pots.

Species are identified utilizing molecular biology methodologies, specifically through the amplification and sequencing of the 16S rDNA region. DNA is extracted using the DNA Mini Kit (Syngen). The 16S rDNA fragment is amplified via PCR using the 27F and 1492F primers, [2] using DREAMTAQ HS GREEN MASTER MIX (Thermo Scientific) reagents. PCR conditions: initial denaturation in 95°C for 3 min; 35 cycles consisting of denaturation in 95°C for 30 s; primer annealing in 60°C for 30 s and elongation in 72°C for 90 s; final elongation in 72°C for 5 min. PCR products were analyzed by agarose gel electrophoresis. The PCR products were sequenced using the 27F and 1492R primers. Nucleotide sequences were analyzed using Genious Prime and compared with sequences from the NCBI database (www.nebi.nlm.nih.gov) utilizing the BLASTn algorithm. The results of the molecular identification are presented in Table 2.

**Table 2. The results of the molecular identification of bacteria used in the inoculum.**

| **#** | **Strain** | **Name of the identified strain** | **Reference sequence (NCBI)** | **Similarity** |
|---|---|---|---|---|
| **1.** | UNIJAG.PL.OP280 | *Chryseobacterium lathyri* | Chryseobacterium lathyri NR_115853 | 1333/1339 (99%) |
| **2.** | UNIJAG.PL.OP290 | *Lysinibacillus fusiformis* | Lysinibacillus fusiformis NR_112628 | 1370/1370 (100%) |
| **3.** | UNIJAG.PL.OP287 | *Bacillus cereus* | Bacillus cereus NR_115714 | 1374/1374 (100%) |
| **4.** | UNIJAG.PL.OP300 | *Pseudomonas protegens* | Pseudomonas protegens NR_114749 | 1348/1349 (99%) |

Surprisingly, it has been discovered that the formulated preparation expedites plant growth, enhancing both the green part and root biomass, negating the necessity for additional supplementation. Surprisingly, it was also found that combining the biopreparation with fertilization yields superior plant biomass outcomes compared to standalone fertilization. The observed results highlight the potential to substantially curtail plant fertilization during seedling production due to the efficacy of the biopreparation, thereby reducing production costs. In seedling production, root system development is of extreme importance. The employed biopreparation notably augmented root biomass in the examined plants. The adoption of the biopreparation in industrial production has the potential to wholly negate the demand for supplemental preparations, which are employed as foliar applications of agrochemical compounds, targeting root enhancement. Biotization of plants via the inoculum necessitates only two soil applications, eliminating other treatments in seedling production.

The described inoculum, as derived from the inventive methodology, boasts significant commercialization potential, and may be used in Brassicaceae plant cultivation. Due to the surprising properties of the inoculum in the aspect of enhancing plant growth parameters, it can serve as a substitute to conventional inorganic salt-based fertilizers or function as a co-supplement.

These surprising properties of the resulting inoculum may, without being bound by any theory, be attributed to the proficient colonization of plant roots by the bacterial strains within the inoculum. This colonization could potentially modulate the plant's water-mineral balance. Such modulation amplifies the plant's absorption of water and minerals. The inoculum's endophytic bacteria may further modulate the plant's phytohormonal balance, consequently accelerating the growth and augmenting the biomass during the plant's nascent growth stages. Furthermore, the obtained inoculum maintains its activity throughout the seedling's initial growth phase, ensuring sustained enhancement of plant growth parameters.

### Brief Description of the Figures

Figure 1 compares the fresh plant mass between inoculated (T4) and non-inoculated plants. A denotes plants treated with fertilizer at a dose of 0; B denotes plants treated with fertilizer at a substrate concentration of 0.5% w/w; C denotes plants treated with fertilizer at a substrate concentration of 1.0% w/w. Differences that are statistically significant between inoculated and non-inoculated plants at the alpha significance levels of 0.05, 0.01, and 0.005 are designated with *, **, and ***, respectively.
Figure 2 illustrates the effect of individual bacterial strains from the T4 inoculum composition on the growth of Brassicaceae plants, as measured by changes in the fresh shoot mass.
Figure 3 portrays the influence of individual bacterial strains from the T4 inoculum composition on Brassicaceae plant growth, as measured by changes in the leaf area.
Figure 4 depicts the impact of individual bacterial strains from the T4 inoculum composition on Brassicaceae plant growth, as measured by changes in the fresh root mass.
Figure 5 delineates the effect of individual bacterial strains from the T4 inoculum composition on Brassicaceae plant growth, as measured by changes in the dry root mass.
Figure 6 demonstrates the effect of the evaluated T3 inoculum composition on the fresh mass of select plant species.

Selected inocula for plant biotization, formulated and evaluated according to the inventive method, are further detailed in subsequent embodiments.

### Example 1. Preselection of Microorganisms for Inoculum Composition

The objective was to formulate a microbial consortium capable of enhancing plant growth and improving the qualitative parameters of Brassicaceae plants. Endophytic bacteria were isolated from the naturally occurring populations of the sand rock-cress *(Arabidopsis arenosa* (L.) Lawalrée). Identification of the bacteria was achieved through nucleotide sequencing of the 16S rDNA region. Subsequent selection incorporated only those microorganisms which were identified to the species level. Given the assumption that bacteria within the consortium should exhibit a wide spectrum of biotization properties, they were evaluated for traits such as indoleacetic acid (IAA) synthesis, siderophores production, and the capability to solubilize organic P. Furthermore, the observed synergistic effects of similar microorganisms (e.g., mycorrhizal microorganisms, endophytes) on plant growth, as previously documented by the Plant-Microorganism Interactions Lab of the Malopolska Centre of Biotechnology at Jagiellonian University (data not shown), was leveraged. Consistent with prior observations, the inclusion of such microorganisms in the formulation augmented their influence on plant growth compared to preparations containing individual microorganisms. Incorporating a consortium of microorganisms in the inoculum broadened the spectrum of plant species that responded to the vaccine. This approach also mitigated potential incompatibilities between select bacterial strains and specific host species. The specific microbial traits were analyzed using the following methods:
1. IAA synthesis: assayed using HPLC-MS + colorimetry with the Salkowski reagent; (Gang et al. 2019)
2. siderophores production: assayed using the method described by Schwyn and Neilands (1987) with Chrome Azurol S (CAS);
3. P dissolution: detected by observing the light halos surrounding bacterial colonies due to the solubilization of calcium phosphate on solid media in Petri dishes, as per the method described in Pikovskaya (1948).

The results are listed in Table 3. In this table, the '+' symbol represents the ability of a microorganism to perform a particular reaction, while the negative '-' symbol indicates an inability to perform said reaction.

**Table 3. Ability of selected microorganisms to synthesize IAA, produce siderophores and P utilization.**

| | **Tested parameter** | | |
|---|---|---|---|
| **Isolate** | **IAA synthesis** | **Siderophores** | **P utilization** |
| *Brevibacillus nitrificans* OP247 | + | - | + |
| *Pseudomonas rhizosphaere* OP237 | + | + | + |
| *Pseudomonas lutea* OP193 | + | + | + |
| *Pantoea agglomerans* OP198 | + | + | + |
| *Acidovorax valerianellae* OP253 | + | + | + |
| *Sporobolomyces ruberrimus* OP177 | + | + | - |
| *Psychrobacillus psychrodurans* OP200 | + | - | + |
| *Pseudomonas rhodesiae* OP244 | + | + | + |
| *Aureobasidium pullulans* OP1164 | + | - | + |
| *Chryseobacterium lathyri* OP280 | + | + | + |
| *Lysinibacillus fusiformis* OP290 | + | + | + |
| *Bacillus mycoides* OP287 | + | + | - |
| *Pseudomonas protegens* OP300 | + | + | + |
| *Pseudomonas koreensis* OP519 | - | - | - |
| *Pseudomonas brassicacearum* OP563 | - | - | + |
| *Pseudomonas helmanticensis* OP349 | - | - | - |
| *Lelliottia nimipressuralis* OP369 | - | - | - |
| *Lysinibacillus fusiformis* OP267 | - | - | - |
| *Lelliottia amnigena* OP458 | - | - | - |
| *Bacillus safensis* OP506 | + | - | - |
| *Enterobacter ludwigii* OP479 | + | - | - |
| *Delftia acidovorans* OP538 | + | - | - |
| *Solibacillus silvestris* OP364 | + | - | - |

Based on the test results, the following microorganism consortium compositions were formulated, which were employed in growth acceleration and augmentation of production parameters in selected species/varieties of Brassicaceae plants:
**T1:** *Brevibacillus nitrificans* OP247, *Pseudomonas rhizosphaere* OP237, *Pseudomonas lutea* OP193 , *Acidovorax valerianellae* OP253, *Sporobolomyces ruberrimus* OP177
**T3:** *Psychrobacillus psychrodurans* OP200, *Pseudomonas rhodesiae* OP244, *Aureobasidiung pullulans* OP1164
**T4:** *Chryseobacterium lathyri* OP280, *Lysinibacillus fusiformis* O290, *Bacillus cereus* OP287, *Pseudomonas protegens* OP300

### Example 2. Inoculum Preparation

For the inoculum preparation, the bacterial strains used were *Chryseobacterium lathyri* (UNIJAG.PL.OP280), *Lysinibacillus fusiformis* (UNIJAG.PL.OP290), *Bacillus cereus* (UNIJAG.PL.OP287), and *Pseudomonas protegens* UNIJAG.PL.OP300*)*. Bacterial strains were cultured in sterilized nutrient agar (NA) liquid medium, comprised of 5 g/L of peptone, 3 g/L of meat extract, and 15 g/L of agar. Cultures were housed in 250 mL conical flasks sealed with cellulose stoppers, and secured with aluminum foil, and incubated for 72 hours at 30°C with shaking at 180 rpm. Post-incubation, the cultures exhibited an optical density (OD600) of 1.8 ± 0.4 Abs. The cultures were then centrifuged at 5000 g for a duration of 5 minutes, the supernatant was decanted, and the bacterial precipitate was washed using a 0.9% NaCl solution. The washing protocol was executed twice: the precipitate was resuspended in the 0.9% NaCl solution, vortexed for 30 seconds, centrifuged, and the supernatant subsequently decanted. The resulting precipitate was resuspended in 40 mL of a 0.9% aqueous NaCl solution, with each culture processed separately. Upon acquiring bacterial suspensions in saline, they were mixed and then further diluted to a final volume of 1 L using a 0.9% aqueous NaCl solution in a volumetric flask.

For the preferred vaccine embodiment, the recorded OD value of 1.8 Abs confirmed the successful culture of the microorganisms.

### Example 3. Confirmation of the Vaccine's Impact on Plant Biomass under Laboratory Conditions

The suspension detailed in Example 1 was utilized for plant inoculation. For each pot compartment within the multi-pot system, a blend of non-sterile universal plant soil and tap water was prepared at a ratio of 1:2. A single plant seed was then introduced to each pot. 1 mL of the inoculum was dispensed into each pot. For control, pots were treated with 1 mL of a 0.9% aqueous NaCl solution both at the time of sowing and on the 10th day of cultivation. Inoculation was reiterated on the 10th day, with 1 mL of inoculum being added to each respective plant pot. All plants were housed within a plant grow box with the following cultivation parameters: a photoperiod of 16 h, light intensity at 190 µmol · m² · s⁻¹, temperature of 24/19°C, and humidity at 70%.

It was confirmed that the inoculum imparted a favorable influence on the fresh biomass of cultivated plant species, specifically mizuna, violet cauliflower, kale, Chinese cabbage, napa cabbage, and white cabbage showcased an enhancement in fresh plant matter by roughly 40% vs control. The green cauliflower exhibited a modest enhancement, approximately 10% vs control.

### Example 4. Verification of the Inoculum's Efficacy on Growth Parameters in Semi-Technical Scale Cultivation Conditions and Comparison with Inorganic Salts-Based Fertilization

### Stage I

Brassicaceae seedling growth was assessed. For the experiment, the followint plants were used: ' Kamienna Glowa' white cabbage (PNOS w Ozarowie Mazowieckim Sp. z o.o.), 'Koda' red cabbage (W. Legutko Przedsi 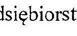 biorstwo Hodowlano-Nasienne Sp. z o.o.), 'Verde di Macerata' green cauliflower (PlantiCo Hodowla i Nasiennictwo Ogrodnicze Zielonki Sp. z o.o.), 'Di Sicilia Violetto' violet cauliflower W. Legutko Przedsi 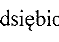 biorstwo Hodowlano-Nasienne Sp. z o.o.), 'Halbhoher Grüner Krauser' kale (W. Legutko Przedsi 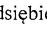 biorstwo Hodowlano-Nasienne Sp. z o.o.), 'Athena' arugola (Tozer Seeds Ltd), 'Manoko' F1 napa cabbage (PlantiCo Hodowla i Nasiennictwo Ogrodnicze Zielonki Sp. z o.o.), 'Pak-choi' Chinese cabbage (W. Legutko Przedsi 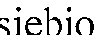 biorstwo Hodowlano-Nasienne Sp. z o.o.), and mizuna (Torseed S.A.). The T4 bacterial vaccine was employed for plant inoculation, while non-inoculated plants were utilized as controls.

The experiment was executed in triplicate. A single replicate comprised half of a 96-cell VEFI seedling tray, equivalent to 48 cells/plants. These trays, black in color, featured conical cells with a volume of 53 cm³ each. Once filled with the Florabalt Growing medium (Floragard Vetriebs GmbH, Oldenburg, Germany), which consists of white Baltic peat deacidified with lime and supplemented with primary and trace elements, the trays were positioned on production tables. Seeds were sown into these trays (with one seed for each tray cell, except for arugula which required thinning after planting to achieve a single seedling per cell) and then overlaid with a thin layer of peat substrate.

Artificial lighting was applied during morning and evening hours using HPS sodium lamps (Elektro Valo Oy, Lady bird, Netafim Ltd.), positioned 100 cm above the tables, resulting in a 14-hour lighting cycle with a radiation intensity (PPFD) of 200 µmol · m⁻² · s⁻¹. A natural light was used for any irradiation exceeding 200 W/m². The temperature was maintained between 22-24°C until emergence, and following emergence (3-4 days post-sowing), it was maintained between 18-20°C (as average daily air temperature). The condition of the seedlings and substrate moisture were observed daily, with watering carried out using a lance for small droplet delivery. Post-sowing and substrate hydration, the initial vaccine inoculation was executed, followed by a second inoculation a week later. Three weeks from sowing, a foliar application using the 'Kristalon Zielony 18-18-18' fertilizer (YARA Poland Sp. z o.o.) was implemented at a 1% solution concentration, dispensing 60 mL of water/solution for every 96-cell tray.

Subsequent measurements and analyses of the plants were executed as follows: 1) Sequential leaf count: Leaves with a minimum length of 5 mm were counted, with the count being performed on the same, distinctly labeled plants; 15 plants per subject, and 5 plants from individual repetitions were chosen for this. The counting procedure was repeated three times, each interval a week apart, beginning two weeks post-sowing.2) Optimal Phase Leaf Count: At an approximate duration of 4-5 weeks post-sowing, during a phase deemed optimal for planting, all leaves on the plants with a minimum length of 5 mm were counted.3) Concurrent Evaluation of Fresh and Dry Biomass: For the above-ground portions and the roots of the plants, a concurrent assessment of fresh and dry biomass was conducted. A total of 15 plants per each subject were chosen at random. Each plant and its parts parts were weighed using a laboratory balance (Sartorius A120S, Sartorius AG, Goettingen, Germany) after removal of any peat substrate from the roots. The dry biomass measurement was determined following the desiccation of samples at 92-95°C.4) Stem Diameter: The stem's diameter measured between the cotyledons and the first true leaves, was ascertained utilizing an electronic caliper.5) Subsequent to the weighing, the most visually prominent leaves (a total of 15) were cut-off from the plants and subjected to image analysis using WinDIAS, Delta-T Devices, UK, to discern key leaf parameters, including but not limited to area, perimeter, length, width, and shape factor.

A statistically significant positive impact of the vaccine on the plant growth parameters was discerned. Specifically, this advantageous effect was noted for the ensuing parameters when compared with the control (non-inoculated plants):
Mizuna: leaf area (approx. 6%), leaf perimeter (approx. 7%), fresh mass of the above-ground parts (approx 7%).

Violet cauliflower: leaf area (approx. 16%), leaf perimeter (approx. 9%), leaf length (approx. 8%), fresh mass of the above-ground parts (approx 20%).

Green cauliflower: leaf perimeter (approx. 7%), fresh mass of the above-ground parts (approx 23%).

Kale: leaf area (approx. 10%), leaf perimeter (approx. 6%), leaf length (approx. 12%), fresh mass of the above-ground parts (approx 9%).

Napa cabbage: leaf area (approx. 15%), leaf length (approx. 7%), fresh mass of the above-ground parts (approx 28%).

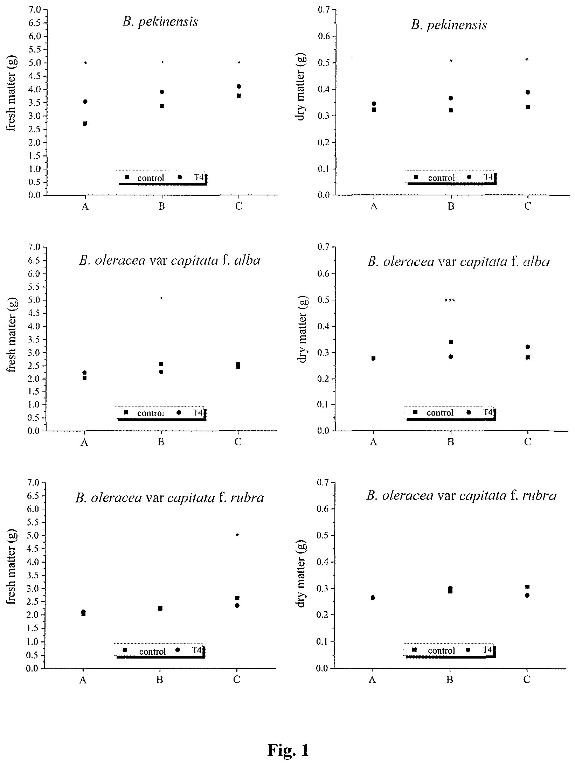

Additionally, in a comparative assay with conventional fertilization employing inorganic salts in cabbage (napa cabbage, white cabbage, and red cabbage), a beneficial inoculum impact (Fig. 1) was observed in:
- Napa cabbage: fresh mass of non-fertilized plants, plants fertilized with 0.5% w/w and 1% w/w of salt in the substrate; dry mass of plants fertilized with 0.5% of salt in the substrate.
- White cabbage: fresh mass of plants fertilized with 0.5% w/w of salt in the substrate; dry mass of plants fertilized with 0.5% of salt.
- Red cabbage: fresh mass of plants fertilized with 1.0% w/w of salt in the substrate.

### Stage II

The effect of substituting standard foliar fertilizers with the inocula from Stage I was assessed. The plant subjects encompassed: napa cabbage, white cabbage, and red cabbage, which were inoculated with the T4 inoculum; and napa cabbage, green cauliflower, arugola, white cabbage, red cabbage which served as controls. Inoculations were executed immediately post-sowing and subsequently a week later. Each experimental set, which comprised a Brassicaceae plant from both the vaccinated and control groups, received fertilization treatments at full foliar fertilizer concentration (100% subjects), half the specified concentration (50% subjects), and distilled water (0% subjects). 2.5 weeks post-sowing, plants were subjected to fertilization with the 'Kristalon Zielony 18-18-18' foliar fertilizer (YARA Poland Sp. z o.o.) (1%, 0.5% and 0% solution, volume of solution/water: 60 mL per each 96-cell tray). The experiment was replicated thrice, with each replicate comprising 32 individual plants.

Microclimatic conditions and cultivation protocols during this experimental phase mirrored those delineated in Stage I. Subsequent measurements and analyses, as defined, for Stage II encompassed: leaf enumeration at harvest (occurring 30-35 days post-sowing), evaluation of fresh and dry biomass of the above-ground parts, and leaf parameters (analysed with the WinDIAS image analysis system).

Surprisingly, statistical evaluations elucidated that the vaccine's impact parallels that of conventional fertilizers. No statistically significant differences were observed between plants treated with the inoculum and those fertilized using inorganic salts, based on the previously defined matrix of variants. However, exceptions were noted for fresh biomass of red cabbage treated with a 1% salt solution and white cabbage treated with a 0.5% salt solution, where the inorganic salt exhibited marginally superior results. Nevertheless, the vaccine demonstrated efficacy across most scenarios, promoting enhanced biomass production when utilized either independently or in co-supplementation (simultaneously mitigating chemical usage), and the inoculum implementation requires optimization predominantly during the initial phase.

Surprisingly, the advantageous effects were exclusively attributed to the T4 inoculum. In contrast, other evaluated vaccines, such as T3, or individual strains from the T4 inoculum, manifested significantly diminished effects no effects at all.

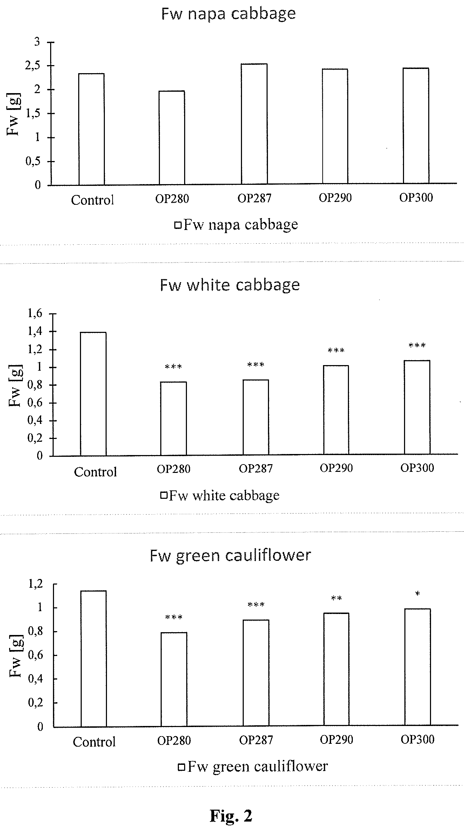

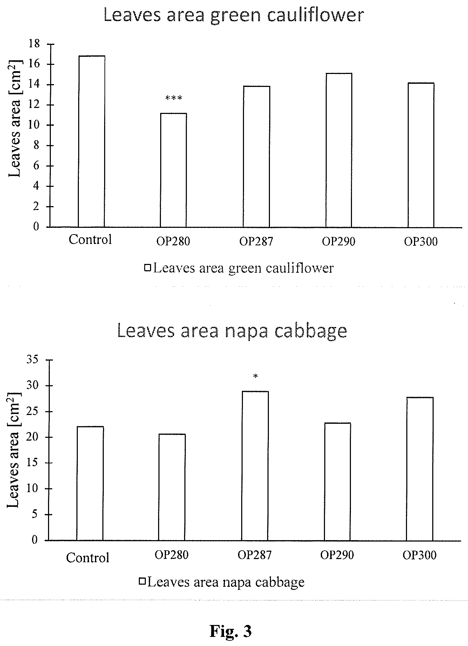

Fig. 2-5 illustrate the influence of individual bacterial strains present in the T4 inoculum composition on the growth of Brassicaceae plants. None of the T4 inoculum components showcased a beneficial impact on fresh shoot biomass. For cabbage and green cauliflower, a notable reduction in plant mass was identified in inoculated specimens when compared to controls (Fig. 2). The OP280 strain adversely impacted the leaf surface area in green cauliflower. The 287 strain positively influenced the leaf surface area in napa cabbage.

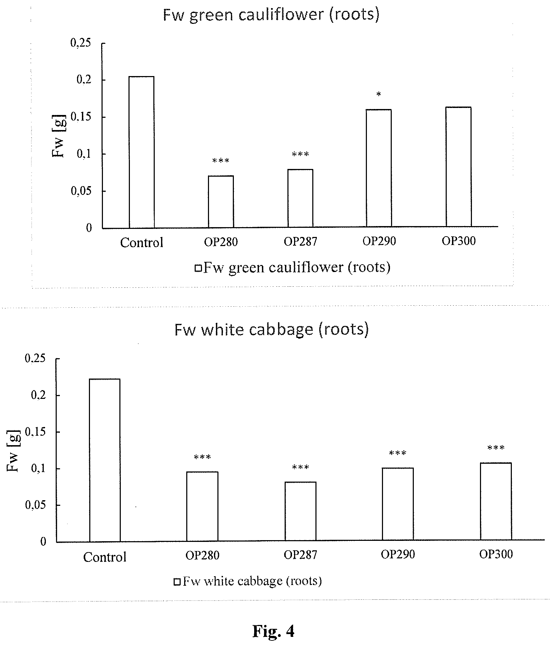

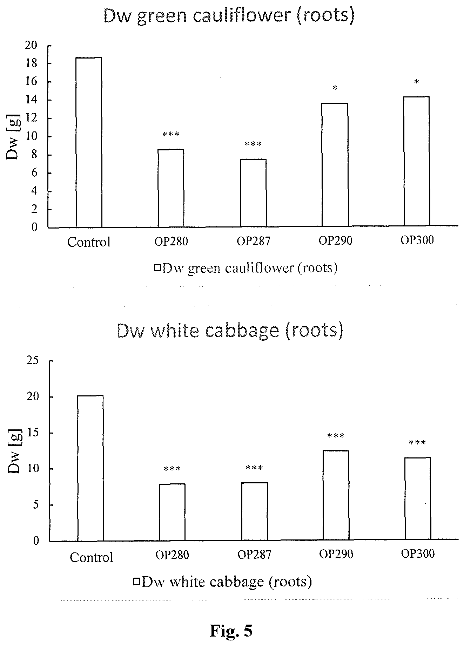

No component of the T4 inoculum presented a beneficial effect on fresh root biomass. A substantial majority of strains curtailed the fresh root biomass in both green cauliflower and cabbage (Fig. 4). All bacterial strains exerted a negative effect on the dry biomass of green cauliflower and cabbage (Fig. 5).

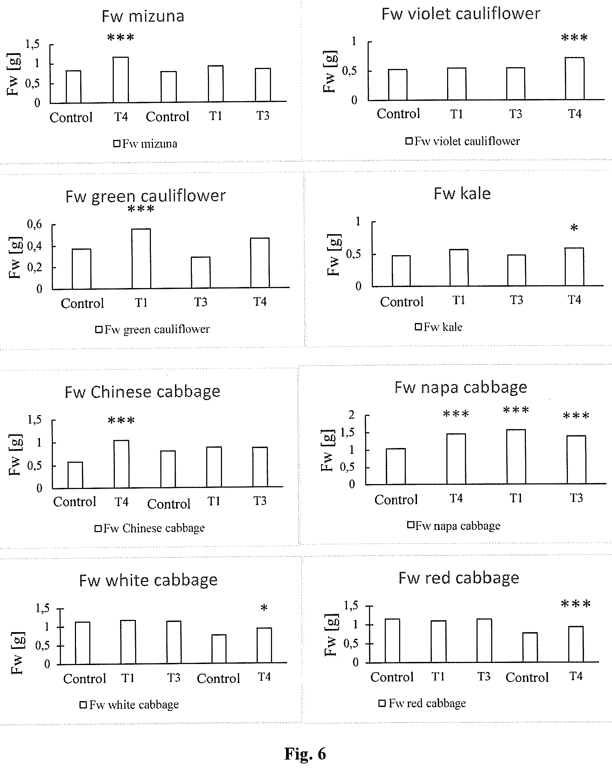

Fig. 6 illustrates experimental findings, indicating the absence of a beneficial impact of the T3 vaccine on the fresh biomass of selected plant species.

### References:

[1] J. Poveda, R. Hermosa, E. Monte, C. Nicolás, Trichoderma harzianum favours the access of arbuscular mycorrhizal fungi to non-host Brassicaceae roots and increases plant productivity, Sci. Rep. 9 (2019) 1-11. https://doi.org/10.1038/s41598-019-48269-z.
[2] S. Turner, K.M. Pryer, V.P.W. Miao, J.D. Palmer, Investigating deep phylogenetic relationships among cyanobacteria and plastids by small subunit rRNA sequence analysis, J. Eukaryot. Microbiol. 46 (1999) 327-338. https://doi.org/10.1111/j.1550-7408.1999.tb04612.x.

## Claims

1. A bacterial composition comprising the following strains: *Chryseobacterium lathyri* deposited in PCM under No. B/00412, *Lysinibacillus fusiformis* deposited in PCM under No. B/00414, *Bacillus cereus* deposited in PCM under No. B/00413, and *Pseudomonas protegens* deposited in PCM under No. B/00415

2. A inoculum for Brassicaceae plant biotization containing the composition defined in claim 1.

3. The inoculum according to claim 2 **characterized in that** the inoculum is intended for accelerating the growth of Brassicaceae plants, preferably for increasing the mass gain of the green parts or roots of the plants.

4. The inoculum according to claim 2 **characterized in that** it is in the form of an aqueous bacterial suspension.

5. Bacterial strain selected from: *Chryseobacterium lathyri* deposited in PCM under B/00412, *Lysinibacillus fusiformis* deposited in PCM under B/00414, *Bacillus cereus* deposited in PCM under B/00413, and *Pseudomonas protegens* deposited in PCM under B/00415.

## Patentansprüche

1. Bakterielle Zusammensetzung umfassend die folgenden Stämme: *Chryseobacterium lathyri*, hinterlegt in PCM unter Nr. B/00412, *Lysinibacillus fusiformis*, hinterlegt in PCM unter Nr. B/00414, *Bacillus cereus*, hinterlegt in PCM unter Nr. B/00413, und *Pseudomonas protegens*, hinterlegt in PCM unter Nr. B/00415

2. Inokulum für die Biotisierung von Brassicaceae-Pflanzen, enthaltend die in Anspruch 1 definierte Zusammensetzung.

3. Inokulum nach Anspruch 2, **dadurch gekennzeichnet, dass** das Inokulum dazu bestimmt ist, das Wachstum von Brassicaceae-Pflanzen zu beschleunigen, bevorzugt die Massenzunahme der grünen Teile oder Wurzeln der Pflanzen zu erhöhen.

4. Inokulum nach Anspruch 2, **dadurch gekennzeichnet, dass** es in der Form einer wässrigen Bakteriensuspension ist.

5. Bakterienstamm, ausgewählt aus: *Chryseobacterium lathyri*, hinterlegt in PCM unter B/00412, *Lysinibacillus fusiformis*, hinterlegt in PCM unter B/00414, *Bacillus cereus*, hinterlegt in PCM unter B/00413, und *Pseudomonas protegens*, hinterlegt in PCM unter B/00415.

## Revendications

1. Composition bactérienne comprenant les souches suivantes : *Chryseobacterium lathyri* déposé auprès de la PCM sous le n° B/00412, *Lysinibacillus fusiformis* déposé auprès de la PCM sous le n° B/00414, *Bacillus cereus* déposé auprès de la PCM sous le n° B/00413, et *Pseudomonas protegens* déposé auprès de la PCM sous le n° B/00415

2. Inoculum pour la biotisation de plante Brassicaceae contenant la composition définie dans la revendication 1.

3. Inoculum selon la revendication 2 **caractérisé en ce que** l'inoculum est destiné à accélérer la croissance des plantes Brassicaceae, de préférence pour augmenter le gain de masse des parties vertes ou des racines des plantes.

4. Inoculum selon la revendication 2 **caractérisé en ce qu'**il est sous la forme d'une suspension bactérienne aqueuse.

5. Souche bactérienne choisie parmi : *Chryseobacterium lathyri* déposé auprès de la PCM sous le n° B/00412, *Lysinibacillus fusiformis* déposé auprès de la PCM sous le n° B/00414, *Bacillus cereus* déposé auprès de la PCM sous le n° B/00413, et *Pseudomonas protegens* déposé auprès de la PCM sous le n° B/00415.
